Europäisches Patentamt

European Patent Office

Office européen des brevets

(19)

(11) EP 0 861 857 A2

(12) EUROPÄISCHE PATENTANMELDUNG

(43) Veröffentlichungstag:
02.09.1998 Patentblatt 1998/36

(51) Int. Cl.⁶: $C08F\ 246/00$, $A61L\ 27/00$, $A61L\ 29/00$

(21) Anmeldenummer: 98100444.3

(22) Anmeldetag: 13.01.1998

(84) Benannte Vertragsstaaten:
AT BE CH DE DK ES FI FR GB GR IE IT LI LU MC
NL PT SE
Benannte Erstreckungsstaaten:
AL LT LV MK RO SI

(30) Priorität: 01.03.1997 DE 19708426
26.09.1997 DE 19742528

(71) Anmelder: Vestolit GmbH
45764 Marl (DE)

(72) Erfinder:
• Jozefowicz, Marcel, Prof.
60260 Lamorlaye (FR)

• Migonney, Véronique, Dr.
95600 Eaubonne (FR)
• Meyer, Heinz Hermann, Dr.
45770 Marl (DE)
• Neu, Thomas, Dr.
48249 Dülmen (DE)
• Stieneker, Axel, Dr.
48151 Münster (DE)
• Anders, Christine, Dr.
45721 Haltern (DE)
• Ottersbach, Peter, Dr.
51570 Windeck (DE)

(54) Formmassen mit antikoagulierenden Eigenschaften, ihre Herstellung und Verarbeitung zu Gegenständen, die in der Medizintechnik Verwendungen finden

(57) Es wird ein Verfahren zur Herstellung von Formmassen mit antikoagulierenden Eigenschaften beschrieben.

Dabei werden hydrophobe Monomere mit hydrophilen, mit biospezifisch wirksamen Funktionen ausgestatteten Monomeren in Dispersion radikalisch copolymerisiert.

EP 0 861 857 A2

**Beschreibung**

Die Erfindung betrifft die Herstellung von Formmassen mit bestimmten bioaktiven Funktionen, spezielle Maßnahmen bei der Verarbeitung dieser Formmassen zu Gegenständen mit oberflächenwirksamen bioaktiven Funktionen sowie die Verwendung dieser Gegenstände in der Medizintechnik, insbesondere im Kontakt mit Blut oder seinen Bestandteilen.

Der Begriff《Bioaktivität》soll hier im weitesten Sinn als Wechselwirkung zwischen bestimmten statistisch angeordneten Funktionen oder Kombinationen solcher Funktionen in synthetisch erzeugten Makromolekülen und komplementären Funktionen natürlicher makromolekularer Systeme verstanden werden.

Die Wechselwirkung zwischen Blut und fremden Oberflächen, beispielsweise Polymeroberflächen, ist weitgehend bekannt; der Kontakt bestimmter im Blut enthaltener Proteine mit diesen fremden Oberflächen führt zu einer kaskadenartigen Aktivierung verschiedener biochemischer Folgereaktionen, die in der sogenannten Komplementaktivierung oder der Blutkoagulation gipfeln. Seit den 50er Jahren wurde in einer Fülle von detaillierten Experimenten der Versuch unternommen, die Wechselwirkung zwischen fremden Oberflächen und Blut so zu beeinflussen, daß bei vorübergehendem oder auf langzeitigem Kontakt die Blutkoagulation sowie nachteilige Reaktionen des Immunsystems wie Entzündungen etc. unterbleiben.

Die Palette der Maßnahmen ist weit gespannt und teilweise widersprüchlich, wie die folgende Übersicht zeigt, vgl. R. Barbucci u.a.《Modifications to Polymer Surfaces to Imporve Blood Compatibility》, S. 119 ff in: Polymeric Biomaterials, S. Dumitriu Herausg., Marcel Dekker Inc., 1994:

- ♦ Einstellung hydrophober oder hydrophiler Oberflächen

  - hydrophobe Oberflächen nach Alkylierung, dadurch bevorzugte Adsorption von Albumin, dadurch Reduzierung der Blutkoagulation
  - Hydrophilierung von Polymeroberflächen mit Polyethylenglykolketten, dadurch verminderte Protein- und Blutplättchenadsorption

- ♦ Erzeugung von Oberflächen mit Zwitterionen- oder Anionenstruktur

  - neutrale Zwitterionenstrukturen, z.B. polymergebundene Phosphorylcholin-Kopfgruppen (EP 0 275 295, 1986, A.A. Durrani)
  - anionische Strukturen bestimmter Konzentration und Zusammensetzung

Die erfindungsgemäßen Maßnahmen folgen dieser letztgenannten Entwicklungsrichtung.

Hier wurde längere Zeit das natürlich vorkommende Heparin, ein Glykosaminglykan mit Carboxyl-, Sulfat- und Aminsulfatgruppen, favorisiert. Es wurde adsorptiv, ionisch oder kovalent an Polymeroberflächen gebunden; man stellte jedoch fest, daß seine Aktivität relativ schnell abklingt und somit die für klinische Langzeitanwendungen erforderliche Stabilität nicht gegeben ist. Eine weitere Entwicklung bestand darin, die als bioaktiv erkannten Funktionen des Heparins, nämlich Carboxylat und Sulfat/Sulfonat, durch naßchemische polymeranaloge Umsetzungen an den Oberflächen einer Auswahl von vernetzten Polymeren oder an gelösten Polymeren zu fixieren, um auf diese Weise ein heparinähnliches Verhalten zu erzeugen.

Hier sind insbesondere die Arbeiten von Jozefowicz, Jozefowicz und Mitarbeitern zu nennen, vgl. EP-A 0 023 854, 0 090 100, 0 094 893, 0 201 378, 0 203 865 und 0 304 377.

Auch mit Hilfe von Energiequellen wie ionisierenden Strahlen oder elektrischen Entladungen können Polymeroberflächen in der oben beschriebenen Weise modifiziert werden, vgl. R. Barbucci u.a. in obigem Zitat, S. 204 ff.

Schließlich besteht auch die Möglichkeit, die bioaktiven an Vinylmonomere gebundenen Funktionen durch Copolymerisation in Polymersysteme einzuführen oder in dünner Polymerschicht an Polymeroberflächen zu binden.

Beispielhaft für diese Entwicklungsrichtung sind Arbeiten von Miller, Sawyer u.a., J. Appl. Polym. Sci. 1970, 14, 257-266, von Sorm, Nespurek u.a., J. Polym. Sci., Polym. Symp. 1979, 66, 349-356 sowie die EP 0 598 486 der MEDTRONIC Inc. vom 14.10.93. In dieser neueren Anmeldung werden Kombinationen der monomeren Acrylsäure und 2-Acrylamido-2-methylpropansulfonsäure, insbesondere im Molverhältnis 2 : 7, beansprucht. Dieses Monomergemisch wird entweder für sich copolymerisiert und anschließend auf Polymeroberflächen aufgetragen, z. B. durch Tauchen in entsprechenden Lösungen; oder es wird in Gegenwart von Cer-Ionen direkt auf Polymeroberflächen gepfropft.

Allen genannten Verfahren, sofern sie sich auf feste Polymere und deren Oberflächen beziehen, ist gemeinsam, daß die bioaktiven Funktionen in einem weiteren Verarbeitungsschritt auf bereits ausgeformten Gegenständen fixiert werden. Die Nachteile solcher Verfahren liegen auf der Hand:

- Die in der Humanmedizin unabdingbare, reproduzierbare Einstellung identischer bioaktiver Oberflächen, z.B. für eine Serienfertigung von Kathetern und Blutbeuteln, ist auf diese Weise nicht zu gewährleisten.
- Die nachträgliche Modifizierung ist aufwendig bezüglich Zeit und Materialaufwand (Ökonomie und Ökologie).
- Die Wiederverwendbarkeit (Recycling) solcher oberflächenmodifizierter Gegenstände ist nicht möglich, weil nach einem erneuten Umschmelz- und Verarbeitungsschritt nicht die gleichen bioaktiven Oberflächen vorliegen werden.

Diese Nachteile konnten nun durch ein Verfahren und dessen Verwendung gemäß der Patentansprüche überwunden werden.

Gegenstand der Erfindung ist ein Verfahren zur Herstellung von Formmassen mit antikoagulierenden Eigenschaften, wobei hydrophobe Monomere mit hydrophilen, mit biospezifisch wirksamen Funktionen ausgestatteten Monomeren radikalisch copolymerisiert werden sowie die Verwendung dieser Formmassen in der Medizintechnik.

Es wurde überraschend gefunden, daß durch radikalische Copolymerisation einer bestimmten Auswahl geeigneter Vinylmonomerer Formmassen hergestellt werden können, die von Polymerisatteilchen zu Polymerisatteilchen chemisch homogen mit bioaktiven Funktionen versehen sind und die je nach Konzentration und Kombination der mit den Vinylmonomeren eingebrachten bioaktiven Funktion zu Gegenständen ausgeformt werden können, die vielseitige Verwendung in der Medizintechnik finden.

Darüber hinaus wurde gefunden, daß die Verarbeitung zu Gegenständen mit oberflächenwirksamen bioaktiven Funktionen besondere Maßnahmen erfordert, um diese bioaktiven Funktionen auch nach der Verarbeitung gleichmäßig über die Innen- und Oberflächenbereiche der Gegenstände zu verteilen oder gar an der Oberfläche anzureichern.

Das erfindungsgemäße Verfahren zur Herstellung der beanspruchten Formmassen mit antikoagulierenden Eigenschaften zeichnet sich dadurch aus, daß hydrophobe Monomere mit hydrophilen und biospezifisch wirksamen Funktionen ausgestatteten Monomeren radikalisch copolymerisiert wurden.

Ein besonders vielseitiges Verfahren zur Herstellung der erfindungsgemäßen Formmassen ist die radikalische Copolymerisation. Diese kann in Masse, in Lösung mit anschließender Fällung oder in Dispersion erfolgen. Die Copolymerisation in Dispersion umfaßt alle bekannten Verfahren, bei denen das Monomerengemisch oder Teile desselben in disperser Form in einem flüssigen, rührbaren Medium vorliegen. Dieses Trägermedium ist in der Regel Wasser, kann aber auch eine organische Phase sein. Zu diesen bekannten Verfahren zählen die Suspensionspolymerisation und die Emulsionspolymerisation mit in Wasser dispergierter organischer Phase, aber auch ihre inversen Ausführungsformen, bei denen die wäßrige Phase mit den wasserlöslichen Monomeren in organischer Phase dispergiert ist. Auch die speziellen Techniken der Mikro- und Mini-Emulsionspolymerisation können grundsätzlich zur Herstellung der beanspruchten Formmassen angewendet werden. Des weiteren ist auch eine Lösungs-/Fällungscopolymerisation von Vinylchlorid in Alkylalkoholen wie z. B. Methanol, Ethanol, Propanol oder Isopropanol möglich.

Da die Löslichkeiten und Reaktivitäten der verwendbaren Monomeren recht unterschiedlich sein können, müssen die Polymerisationsreaktionen prozeßrechnerkontrolliert und -gesteuert so ausgeführt werden, daß der Einbau der bioaktiven Funktionen möglichst gleichmäßig in allen Makromolekülen und vollständig erfolgt. Deshalb wird von den an sich bekannten Techniken der dosierten Zugabe von Monomerbausteinen während der Polymerisation (Zulaufverfahren) und von der Polymerisation in Stufen Gebrauch gemacht, wie das später in den Beispielen deutlich gemacht wird.

Die Auswahl der radikalisch copolymerisierbaren Monomeren umfaßt sowohl hydrophobe als auch hydrophile, in der Regel mit bioaktiven Funktionen ausgestattete Monomere. Zu den verwendeten hydrophoben Monomeren zählen vor allem Styrol, Methylmethacrylat und Vinylchlorid. Unter den hydrophilen Monomeren werden solche bevorzugt, die bioaktive Grundfunktionen wie Hydroxyl-, Carboxyl- und Sulfatyl- bzw. Sulfonylgruppen tragen. Dazu zählen die Hydroxyalkyl(meth)acrylate und Mono-$(EO)_n$-Vinylether und -Vinylester, Acrylsäure und Methacrylsäure sowie Maleinsäure und Maleinsäureanhydrid und die davon abgeleiteten Halbester und schließlich Vinylsulfonate im weitesten Sinn, vor allem Vinylarylsulfonate wie Styrolsulfonat.

Darüber hinaus können weitere Monomere eingesetzt werden, die als Umsetzungsprodukte der genannten carboxyl- und sulfonylhaltigen Vinylmonomere mit Aminosäuren oder Purin- bzw. Pyrimidinbasen angesehen werden können. Sie sind aufgrund ihrer Struktur in der Lage, spezifische Bindungen zu Proteinen, Enzymen und lebenden Zellen auszubilden.

Bevorzugt werden die Monomeren in Dispersionen radikalisch copolymerisiert.

Die biospezifisch wirksamen Funktionen der hydrophilen Monomeren können ganz oder teilweise mit weiteren biospezifisch wirksamen Funktionen zu

$M - F_1$
$M - F_2$
$M - F_3$
$M - F_1 - F_4$
wobei M = Rest des hydrophilen Monomeren und

$F_1$ = Alkyl-, Aryl- oder Alkyl/Arylhydroxyl-

$F_2$ = Alkyl-, Aryl- oder Alkyl/Arylcarboxyl-

$F_3$ = Alkyl-, Aryl- oder Alkyl/Arylsulfonyl-

$F_4$ = Alkyl-, Aryl- oder Alkyl/Arylphosphatyl-

substituiert sein.

Die biospezifisch wirksamen Funktionen der Formmassen können auch wie folgt aussehen:

$M - F_2 - S_1$

$M - F_3 - S_1$

$M - F_1 - F_4 - S_1$

$M - F_1 - F_4 - S_2$

$M - F_1 - F_4 - S_3$

Mit $S_1$ = Rest einer Aminosäure, die über $-NH_2$ an Carboxyl-, Sulfonyl- oder Phosphatyl- gebunden ist, wobei die bevorzugten Aminosäure Glutamin, Threonin, Methionin, Cystein, Cysteinsäure, Prolin, Hydroxyprolin, Serin, Tyrosin, Benzoyloxycarbonyl-Lysin, tert.-Butyloxycarbonyl-Lysin, $\varepsilon$-Aminocapronsäure, $\beta$-Alanin, $\gamma$-Amino-n-Buttersäure und substituierte oder unsubstituierte $\delta$-Amino-n-Valeriansäure sind und $S_2$ = N-hydroxiethylierte Purin- oder Pyrimidinbasen oder Zuckerrest-Purin- oder Zuckerrest-Pyrimidinbase und $F_4 - S_3$ = Phospholipid mit z. B. $S_3$ = Cholin bedeuten.

Die Aufarbeitung der Polymerdispersionen erfolgt durch Sprühtrocknung, Fällung, Elektrophorese oder einen anderen Form der Trennung fest-flüssig mit anschließender Trocknung.

Das trockene Pulver wird allein oder in Abmischung mit VC-Homopolymerisaten (E-, S- oder Mikro-S-PVC) und/oder Copolymerisaten mit den für die jeweilige Verarbeitung notwendigen und für die spätere Verwendung zugelassenen Hilfsmitteln wie Stabilisatoren, Gleitsubstanzen, Weichmachern etc. entweder über eine Granulat-Zwischenstufe oder direkt zu Gegenständen für medizinische Anwendungen verarbeitet. Die Formmassen können mit 5 bis 85 Gew-% eines Weichmachers wie Mono- und Diester von Dicarbonsäuren und Hydroxy-Dicarbonsäuren, epoxidierte Naturöle, epoxidierte Fettsäuren und deren Ester, Polyester und Ester der Trimellinsäure abgemischt sein. Bevorzugt werden Dioctylphthalat, Zitronensäureester, epxodierte Soja- und Leinöle und Alkylepoxystearate mit Epoxidgehalten zwischen 1,5 und 10 % eingesetzt.

Diese Ausformung zu Gegenständen mit bioaktiven Oberflächen erfordert besondere Maßnahmen, wie ein einfaches Experiment verdeutlichen soll:

Stellt man z.B. ein Vinylchlorid-Polymer nach einem der oben beschriebenen Verfahren her, löst dieses in Tetrahydrofuran, gießt die Lösung in eine flache Petrischale und läßt das Lösungsmittel verdunsten, so erhält man eine Folie. Wird nun an dieser Folie mit Hilfe geeigneter analytischer Methoden senkrecht zur Oberfläche die Verteilung der Sulfonatfunktionen gemessen, so findet man überraschend eine inhomogene Verteilung, nämlich eine Verarmung an der von Luft kontaktierten Oberseite und eine Anreicherung an der dem Glas zugewendeten Unterseite.

Ähnliche Ergebnisse erhält man auch bei der Verarbeitung, z. B. der Extrusion zu Schläuchen für den Einsatz als Katheter. In diesem Fall sind beide Oberflächen, die Innen- und die Außenseite, an bioaktiven Funktionen verarmt.

Erst wenn die formgebenden Werkzeuge in den Bereichen, in denen mit beginnender Abkühlung die Ausformung der homogenen Schmelze zu Gegenständen stattfindet, mit polaren Oberflächen ausgerüstet sind, kommt man zu dem gewünschten Ergebnis: Die bioaktiven Funktionen sind gleichmäßig in den Innen- und Außenbezirken des Gegenstandes verteilt oder sogar an deren Grenzflächen angereichert.

Die Ausrüstung der Oberflächen der formgebenden Werkzeuge sollte an der Kontaktfläche eine Oberflächenspannung > 50 mN/m aufweisen und kann auf verschiedene Weisen erfolgen:

♦ Beschichtung oder Auskleidung mit wärmebeständigen Werkstoffen wie Metallegierungen oder Kunststoffen (vernetzt oder unvernetzt) wie z. B. Phenol/Formaldehyd-Kondensaten, Nylon-4, Nylon-6, Nylon-6,6 oder keramischen Stoffen

♦ Erzeugen eines kontinuierlichen Wasser/Wasserdampf-Gleitfilms.

Der Nachweis der Bioaktivität, speziell des antikoagulierenden Verhaltens solcher Gegenstände, z. B. von Schläuchen, wird wie folgt durchgeführt:
Die Schläuche werden in einer Umlaufapparatur (15 ml/min)

- 3 Waschzyklen zu 3 Stunden mit 1,5 molarer NaCl bei 90 °C

- 3 Waschzyklen zu 3 Stunden mit Wasser bei Raumtemperatur
- 3 Waschzyklen zu 3 Stunden mit Michaelis Puffer bei Raumtemperatur unterworfen.

Anschließend werden Absorptionsexperimente mit radioaktiv markiertem Human Serum Albumin, [125]J-HSA, durchgeführt, um die wirksame Oberfläche zu bestimmen. Dazu werden Schläuche wiederum in einer Umlaufapparatur (15 ml(min) bei Raumtemperatur 40 min lang einer [125]J-HSA Pufferlösung (3 mg/ml) ausgesetzt. Anschließend stellt man an den leeren Schläuchen die Radioaktivität fest. Die spezifische Menge an adsorbiertem Albumin in mg/cm$^2$ geometrischer Oberfläche ergibt sich dann aus der resultierenden Radioaktivität in cpm (counts per minute) dividiert durch die spezifische Radioaktivität des [125]J-HSA in cpm/mg, weiterhin dividiert durch die spezifische Oberfläche in cm$^2$.

Schließlich wird die Thrombin (T) - Antithrombin (AT)-Wechselwirkung in Gegenwart der Schlauchinnenflächen bestimmt. Als Maß für diese Wechselwirkung wird die Geschwindigkeitskonstante $k_{app}$ in ml/u $\cdot$ min $\cdot$ 10$^{-3}$ (u = units = Einheiten) in der Reaktion

$$T + AT \xrightarrow{\phantom{aa}k_{app}\phantom{aa}} T\text{-}AT \qquad -d(T)/dt = k_{app} \cdot [T] \cdot [AT]$$

herangezogen.

Dazu wird AT in Michaelis Puffer (0,2 u/ml) in einer Umlaufapparatur (15 ml/min) während 30 min umgepumpt. Dann wird T (1.000 u/ml) in 0,25 ml-Inkrementen bis zu einem Gesamtvolumen von 5 ml zur zirkulierenden Lösung hinzugefügt. Die T-AT Reaktion wird nun nach Entnahme von 0,12 ml Lösung in 5 Minutenintervallen durch T-Bestimmung verfolgt. Trägt man 1/T gegen die Zeit auf so erhält man eine Gerade mit der Steigung $k_{app}$.

Aufgrund der beschriebenen vielseitigen Verfahren zur Herstellung von Formmassen und Gegenständen mit variabler Konzentration und Kombination bioaktiver Funktionen ergibt sich eine große Vielfalt von Anwendungsmöglichkeiten für die resultierenden Gegenstände.

Das erfindungsgemäß erhaltene Pulver kann sowohl in gelöster Form als auch in fester Form als Coatingmittel mit antikoagulierender Wirkung verwendet werden. Die zu Gegenständen ausgeformten Formmassen sind dank der einstellbaren bioaktiven Oberflächen für den medizinischen und chirurgischen Gebrauch im weitesten Sinne einsetzbar, z. B. als Schläuche, Katheter, Blutbeutel, Folien, Fäden, kardiovaskuläre Prothesen etc.

Ein weiteres Feld erschließt sich auch für die Verwendung als stationäre Phase, kompakt oder in dünner Schicht auf einem mechanisch stabilen Träger, in allen Arten chromatographischer Trennverfahren zur Reindarstellung bestimmter Blutbestandteile, Enzyme, Co-Enzyme oder enzymatischer Komplexe. Schließlich können die beanspruchten Systeme auch für verschiedene biologische Analysenverfahren Verwendung finden, z. B. bei der Elektrophorese, der Immunelektrophorese, RIA, ELISA usw.

Die folgenden Beispiele beschreiben weitere Details der Erfindung.

Herstellung der Formmassen:

Beispiel 1

In einem beheizbaren 250-Liter-Rührreaktor mit Impellerrührer und Pfaudler-Stromstörer werden in einer ersten Stufe 150 Tle. Wasser (alle weiteren Angaben in Tle. beziehen sich auf die gesamte organische Phase = 100 Tle.) und 5 Tle. Na-Sulfonat vorgelegt und auf 77 °C aufgeheizt. Sodann wird eine 2 %ige Lösung des Initiators Ammoniumperoxodisulfat während 8 Stunden zudosiert. In gleicher Weise werden folgende weiteren Monomeren zudosiert:

10 Tle. Methylmethacrylat während 4 Stunden
7,5 Tle. Hydroxypropylmethacrylat während 4 Stunden
7,5 Tle. Maleinsäuremonobutylester während 8 Stunden.

Anschließend werden in einer zweiten Stufe bei 53 °C 150 Tle. Wasser und 70 Tle. Vinylchlorid hinzugegeben, wobei sich ein Gesamtdruck von 6 bar einstellt.

Schließlich wird das Initiatorgemisch Ammoniumperoxodisulfat und Ascorbinsäure so zudosiert, daß die Polymerisationstemperatur nie 56 °C überschreitet.

Nach Druckabfall wird die Polymerisation weitere 60 min fortgeführt. Die resultierende Dispersion wird entgast und zu einem weißen Pulver sprühgetrocknet.

Beispiel 2

In einem weiteren Polymerisationsversuch im gleichen Reaktor werden 5 Tle. Maleinsäuremonobutylester und 85 Tle. Wasser vorgelegt sowie folgende weitere Monomere zudosiert:

5 Tle. Methylmethacrylat während 6 Stunden
5 Tle. Na-styrolsulfonat während 6 Stunden
5 Tle. Hydroxypropylacrylat während 6 Stunden

In einer 2. Stufe folgen 155 Tle. Wasser und 80 Tle. Vinylchlorid.
Alle weiteren Bedingungen sind die gleichen wie in Versuch Nr. 1.

Beispiel 3

In einem weiteren Versuch im gleichen Reaktor werden 155 Tle. Wasser und 40 Tle. Vinylchlorid vorgelegt und folgende weitere Monomeren zudosiert:

7,5 Tle. Hydroxypropylacrylat während 6 Stunden
7,5 Tle. Maleinsäuremonobutylester während 6 Stunden
5,0 Tle. Na-Styrolsulfonat während 6 Stunden

In einer zweiten Stufe folgt die restliche Menge VCl (40 Tle.). Alle Mengenangaben beziehen sich auf 100 Tle. Monomere.
Alle weiteren Bedingungen sind die gleichen wie in Versuch Nr. 1.

Beispiel 4

In diesem Ansatz werden 80 Tle. Vinylchlorid in 211 Tle. Ethanol gelöst und für die Startrektion mit 0,4 Tle. Azobisisobutyronitril (AIBN) vermischt. Nach dem Aufheizen auf 64 °C und dem Start der Reaktion (Beobachtung einer Wärmetönung), werden folgende Comonomere zudosiert:

7,5 Tle. Hydroxypropylmethacrylat in 8 Stunden
7,5 Tle. Maleinsäuremonobutylester in 8 Stunden
5,0 Tle. Na-Styrolsulfonat in 8 Stunden
3,94 Tle. AIBN in 12 Stunden

Nach der Entgasung von Restmonomer wird die VC-Copolymer-Suspension im Sprühtrockner entwässert.
Die Rezepturen der genannten Beispiele und ihre spezifischen antithrombischen Eigenschaften sind in Tabelle 1 zusammengefaßt:

Tabelle 1

| VC-Copolymer-Nr. | | 1 | 2 | 3 | 4 | S-PVC als Nullprobe |
|---|---|---|---|---|---|---|
| 1. Stufe; Vorlage: | VC | | | 40,0 % | 80,0% | 100% |
| | MMBE | | 5,0% | | | |
| | NaSS | 5,0 % | | | | |
| Dosierung: | HPA | | 5,0 % | 7,5 % | | |
| | HPMA | 7,5% | | | 7,5% | |
| | MMA | 10,0% | 5,0% | | | |
| | MMBE | 7,5 % | | 7,5 % | 7,5 % | |
| | NaSS | | 5,0% | 5,0% | 5,0% | |
| 2. Stufe | VC | 70,0% | 80,0 % | 40,0% | | |
| wasserextrahierbare Anteile | | 3,7 % | 13,4% | 9,7 % | 7,6 % | 0,2 % |

EP 0 861 857 A2

Tabelle 1 (fortgesetzt)

| VC-Copolymer-Nr. | | 1 | 2 | 3 | 4 | S-PVC als Nullprobe |
|---|---|---|---|---|---|---|
| spezifische antithrombische Aktivität SA x $10^{-3}$ (U/cm$^2$) | | 5,52 | 20 | 9,15 | 23,4 | 0,2 |

Die Verarbeitung der erfindungsgemäßen Formmassen wird im nächsten Beispiel beschrieben.

Beispiel 5

In einem Laborextruder (Göttfert MEX 30, Einschneckenextruder) wird eine Mischung aus 100 Gewichtsteilen des aus Beispiel 2 erhaltenen Copolymeren mit 70 Gewichtsteilen EDENOL B 316 (Epoxidiertes Leinöl), 30 Gewichtsteilen Dioctylphthalat und 1 Gewichtsteil Ca/Zn-Stearat zu Schläuchen mit 3 mm Innendurchmesser verarbeitet.

Die gewünschten bioaktiven Funktionen sind in noch größeren Mengen auf der Schlauchoberfläche nachzuweisen, wenn das formgebende Werkzeug mit einer polaren Oberfläche als nur V2A-Stahl ausgerüstet wird. Die Oberfläche des formgebenden Werkzeugs bestand in diesem Beispiel aus: 1. V2A-Stahl (als Vergleich), 2. Phenol-Formaldehydharz (〈polares coating〉).

Die Tabellen 2 und 3 zeigen die unterschiedlichen chemischen und physikalischen Eigenschaften der Extrudate. Nach Verarbeitung über einen phenolharzgecoateten Extrusionsdorn findet man im Vergleich zur Extrusion mit reinem V2A-Stahl deutlich mehr Sauerstoff- und Schwefelatome an der Oberfläche und einen wesentlich höheren polaren Anteil an der Oberflächenspannung.

Tabelle 2

| ESCA-Messungen an VC-Copolymer-Schläuchen aus Beispiel 5 | | | |
|---|---|---|---|
| Element | Peak | VC-Copolymer über Metalldorn extrudiert (Atom-%) | VC-Copolymer über Phenolharzdorn extrudiert (Atom-%) |
| C | 1 s | 76,5 | 66,6 |
| O | 1 s | 11,5 | 19,9 |
| Cl | 2 p | 11,0 | 6,7 |
| Na | 1 s | 0,4 | 4,9 |
| S | 2 p | 0,3 | 2,8 |

Tabelle 3

| Messungen der Oberflächenspannung an VC-Copolymer-Schläuchen aus Beispiel 5 | | | | | |
|---|---|---|---|---|---|
| Proben Nr. | Kontaktwinkel | | Oberflächenspannung | | |
| | $H_2O$ [grd] | $CH_2J_2$ [grd] | disperser Anteil [mN/m] | polarer Anteil [mN/m] | Summe [mN/m] |
| VC-Copolymer über Metalldorn extrudiert | 91 | 35 | 41,5 | 0,6 | 42,1 |
| VC-Copolymer über Phenolharzdorn extrudiert | 64 | 60 | 22,4 | 18,0 | 40,4 |

**Patentansprüche**

1. Verfahren zur Herstellung von Formmassen mit antikoagulierenden Eigenschaften,
dadurch gekennzeichnet,
daß hydrophobe Monomere mit hydrophilen, mit biospezifisch wirksamen Funktionen ausgestatteten Monomeren radikalisch copolymerisiert werden.

2. Verfahren nach Anspruch 1,
dadurch gekennzeichnet,
daß als hydrophobe Monomere Vinylverbindungen wie Styrol, Methylmethacrylat und Vinylchlorid eingesetzt werden.

3. Verfahren nach Anspruch 1,
dadurch gekennzeichnet,
daß als hydrophile Monomere Styrol- und/oder (Meth-)Acryl- und/oder Maleinyl- und/oder Vinylether-Verbindungen eingesetzt werden, wie Styrolsulfonat, Hydroxyalkyl-(meth)acrylat, Methacrylsäure, Maleinsäuremonoester, Vinyl-$(EO)_n$-Ether.

4. Verfahren nach den Ansprüchen 1 bis 3,
dadurch gekennzeichnet,
daß die Monomeren in Dispersion oder in Lösung radikalisch copolymerisiert werden, und daß als Dispersionsmedium bzw. als Lösungsmittel Alkylalkohole wie Methanol, Ethanol, Propanol oder Isopropanol, Wasser, Ketone wie Aceton- oder Methylethylketon und Ether wie Tetrahydrofuran, einzeln oder gemischt, Verwendung finden.

5. Verfahren nach den Ansprüchen 1 bis 4,
dadurch gekennzeichnet,
daß die biospezifisch wirksamen Funktionen der hydrophilen Monomeren ganz oder teilweise mit weiteren biospezifisch wirksamen Funktionen zu

$M - F_1$
$M - F_2$
$M - F_3$
$M - F_1 - F_4$
wobei M = Rest des hydrophilen Monomeren und

$F_1$ = Alkyl-, Aryl- oder Alkyl/Arylhydroxyl-
$F_2$ = Alkyl-, Aryl- oder Alkyl/Arylcarboxyl-
$F_3$ = Alkyl-, Aryl- oder Alkyl/Arylsulfonyl-
$F_4$ = Alkyl-, Aryl- oder Alkyl/Arylphosphatyl-

substituiert sind.

6. Verfahren nach den Ansprüchen 1 bis 4,
dadurch gekennzeichnet,
daß die biospezifisch wirksamen Funktionen in den Formmassen wie folgt aussehen;

$M - F_2 - S_1$
$M - F_3 - S_1$
$M - F_1 - F_4 - S_1$
$M- F_1 - F_4 - S_2$
$M - F_1 - F_4 - S_3$
Mit $S_1$ = Rest einer Aminosäure, die über $-NH_2$, an Carboxyl-, Sulfonyl- oder Phosphatyl- gebunden ist, wobei die bevorzugten Aminosäuren Glutamin, Threonin, Methionin, Cystein, Cysteinsäure, Prolin, Hydroxyprolin, Serin, Tyrosin, Berzoyloxycarbonyl-Lysin, tert.-Butyloxycarbonyl-Lysin, ε-Aminocapronsäure, β-Alanin, γ-Amino-n-Buttersäure und substituierte oder unsubstituierte δ-Amino-n-Valeriansäure sind und $S_2$ = N-hydroxyethylierte Purin- oder Pyrimindinbasen oder Zuckerrest-Purin- oder Zuckerrest-Pyrimidinbase und $F_4 - S_3$ = Phospholipid mit z. B. $S_3$ = Cholin bedeuten.

7. Verwendung der Formmassen nach zumindest einem der vorherigen Ansprüche in Abmischung mit VC-Homo- oder/under Copolymerisaten.

8. Verwendung der Formmassen nach zumindest einem der vorherigen Ansprüche in Abmischung mit 5 bis 85 Gew.-% eines Weichmachers wie Mono- und Diester von Dicarbonsäuren und Hydroxy-Dicarbonsäuren, epoxidierte Naturöle, epoxidierte Fettsäuren und deren Ester, Polyester, und Ester der Trimellinsäure.

9. Verwendung der Formmassen nach zumindest einem der vorherigen Ansprüche in Abmischung mit den für die jeweilige Verarbeitung und für die spätere Verwendung zugelassenen Hilfsmittel wie Stabilisatoren, Gleitsubstanzen, Farbstoffen und Pigmenten.

10. Verfahren zur kontinuierlichen oder diskontinuierlichen Ausformung der nach zumindest einem der vorherigen Ansprüche hergestellten Formmassen zu Gegenständen für medizinische Anwendungen, dadurch gekennzeichnet, daß die mit der homogenen Schmelze im Kontakt stehenden formgebenden Werkzeuge an dieser Kontaktfläche eine Oberflächenspannung > 50 mN/m aufweisen

11. Verfahren nach zumindest einem der vorherigen Ansprüche,
dadurch gekennzeichnet,
daß diese Oberflächenspannung bei der Extrusion oder Coextrusion durch eine geeignete Metallegierung, durch einen Wasser-/Wasserdampf-Gleitfilm oder durch einen Polymerbelag aus Nylon-4, Nylon-6, Nylon-6,6 oder ein vernetztes Phenol-Formaldehydharz oder durch eine Keramik-Auflage eingestellt wird.

12. Verwendung der Formmassen nach zumindest einem der vorherigen Ansprüche als Arzneimittel mit antikoagulierender Wirkung.

13. Verwendung der Formmassen nach zumindest einem der vorherigen Ansprüche und der Gegenstände nach zumindest einem der vorherigen Ansprüche für den medizinischen und chirurgischen Gebrauch, z. B. für Rohre, Katheter, Folien oder Fäden und cardiovaskuläre Prothesen.

14. Verwendung der Formmassen nach zumindest einem der vorherigen Ansprüche und der Gegenstände nach zumindest einem der vorherigen Ansprüche als stationäre Phase, kompakt oder in dünner Schicht auf einem mechanisch stabilen Träger, in der Ionenaustausch- und Affinitätschromatographie.

15. Verwendung der Formmassen nach zumindest einem der vorherigen Ansprüche und der Gegenstände nach zumindest einem der vorherigen Ansprüche zur selektiven Abtrennung und Reindarstellung bestimmter Bestandteile des Blutplasmas.

16. Verwendung der Formmassen nach zumindest einem der vorherigen Ansprüche und der Gegenstände nach zumindest einem der vorherigen Ansprüche zur selektiven Abtrennung und Reindarstellung von Enzymen, Co-Enzymen oder enzymatischen Komplexen.

17. Verwendung der Formmassen nach zumindest einem der vorherigen Ansprüche und der Gegenstände nach zumindest einem der vorherigen Ansprüche in Meßsystemen für die biologische Analyse, z. B. RIA, ELISA, der Elektrophorese, der Immunelektrophorese etc.